# EUROPEAN PATENT SPECIFICATION

(11) **EP 3 066 670 B1**
(45) Date of publication and mention of the grant of the patent: **28.06.2017**
(21) Application number: 14787155.2
(22) Date of filing: 21.10.2014
(51) Int. Cl.: G21K 1/10, H05G 1/00, G21K 1/02, A61B 6/00

(54) **X-RAY IMAGING DEVICE WITH FAST SPATIAL MODULATION OF PHOTON FLUX**
RÖNTGENBILDGEBUNGSVORRICHTUNG MIT SCHNELLER RÄUMLICHER MODULATION DES PHOTONENFLUSSES
DISPOSITIF D'IMAGERIE À RAYONS X À MODULATION SPATIALE RAPIDE DU FLUX DE PHOTONS

(30) Priority: 05.11.2013 EP 13191590
(43) Date of publication of application: 14.09.2016
(73) Proprietor: Koninklijke Philips N.V., 5656 AE Eindhoven (NL)
(72) Inventor: BEHLING, Rolf Karl Otto, NL-5656 AE Eindhoven (NL)
(74) Representative: Damen, Daniel Martijn
(86) International application number: PCT/EP2014/072474
(87) International publication number: WO 2015/067461

(56) References cited:
- US-A- 6 061 426
- US-A1- 2008 037 709
- US-A1- 2011 206 259

## Description

### FIELD OF THE INVENTION

The present invention relates to an X-ray imaging device and to a method of operating such X-ray imaging device.

### BACKGROUND OF THE INVENTION

X-ray imaging is generally used in order to obtain information about internal structures of an object of interest. An X-ray imaging device comprises an X-ray source and an X-ray detector arranged at opposite sides of an observation volume in which the object of interest may be arranged during X-ray imaging. X-rays coming from the X-ray source are transmitted through the object. Due to different X-ray absorption properties of various internal structures of the object, information about the internal structure may be obtained based on a local distribution of X-ray intensities detected by the X-ray detector.

In medical X-ray imaging, the object of interest is typically a region of interest (ROI) of a human or animal body. As X-rays may damage living tissue of such body, it is of general interest to minimize an X-ray dose applied to the body during medical imaging.

In most conventional X-ray imaging devices, X-rays are transmitted through the region of interest homogeneously, i.e. an X-ray intensity applied to the ROI is substantially constant throughout the entire cross-section of the ROI. However, the ROI typically comprises various internal body structures having significantly differing X-ray absorption properties. For example, bones within the ROI show relatively strong X-ray absorbance whereas other tissue types such as muscle tissue or lung tissue show significantly lower X-ray absorbance. Accordingly, with homogeneous X-ray intensity being transmitted throughout the entire ROI, problems may arise in that portions of the ROI with structures having low X-ray absorbance are overexposed whereas portions with strong X-ray absorbance are underexposed.

For example, portions comprising mainly soft tissue like lung tissue hardly absorb X-rays such that a high intensity of X-rays is transmitted through these portions and detector pixels of an X-ray detector capturing the transmitted X-rays may be overexposed, i.e. the detector portions come into over-load conditions. In other words, in these soft tissue portions, the ROI is subject to an excessive patient X-ray dose. On the other hand, there are typically portions within the ROI comprising bones or other strongly X-ray absorbing structures. X-rays transmitted through these portions of the ROI are highly attenuated and detector pixels of an X-ray detector capturing X-rays transmitted through these highly X-ray absorbing portions may be underexposed, i.e. pixels of the X-ray detector in such portions receive only a very low X-ray intensity such that in such "starving" detector cells noise may have a significant impact onto a detector signal.

It may therefore be desired to adapt local intensities of X-rays transmitted through a region of interest to the properties of the internal structures comprised throughout this region of interest. In other words, it would be helpful to have localized and dynamic control of a primary photon flux of X-rays wherein this photon flux may be adapted for example upon a detector signal.

Dynamic attenuators have been proposed, but failed to be practical for fast modulation. For example, WO 0225671 describes an X-ray examination device including, inter alia, an X-ray filter having filter elements with an X-ray absorptivity which can be adjusted by controlling a quantity of X-ray absorbing liquid within individual filter elements. However, re-arranging liquid within filter elements is generally time-consuming and only suitable for low frequency image acquisition.

Similarly, US 6,061,426 discloses an X-ray examination device including such X-ray filter and an X-ray collimator with collimator elements which can be switched between an X-ray transmitting state and an X-ray intercepting state by filling these elements with an X-ray intercepting liquid.

US 2011/0206259 A1 discloses a C-arm imaging device wherein the X-ray imaging beam is filtered to provide a selected filtered beam intensity profile for each projection image being obtained. The beam filter comprises a Digital Beam Attenuator including a plurality of wedge pairs or sets of multiple leafs.

US 2008/0037709 A1 discloses a system and method of controlling a spatial distribution of radiation intensity in a beam of radiation comprising a stack of identical periodic grids as radiation absorbing strucutres.

### SUMMARY OF THE INVENTION

There may be a need for an imaging device enabling localized and dynamic control of a primary X-ray photon flux and a method for operating such X-ray imaging device. Particularly, there may be a need for an approach with which the primary X-ray photon flux may be influenced rapidly such that e.g. overexposure or underexposure of portions of an X-ray detector may be prevented during X-ray imaging. Such needs may be met by the subject-matter of the independent claims. Further embodiments of the invention are defined in the dependent claims.

According to a first aspect of the present invention, an X-ray imaging device for imaging a region of interest of an object arranged within an observation volume is proposed. The X-ray imaging device comprises an X-ray source, a controllable transmission arrangement, an X-ray detector and a control device. The X-ray source is adapted for generating at least partially coherent X-radiation. The controllable transmission arrangement comprises an X-ray phase modifying unit, which comprises at least one sub-unit with periodic sub-structures and an X-ray absorbing unit, which comprises at least one sub-unit with periodic sub-structures both of which units being arranged within a beam path of X-rays emitted by the X-ray source one behind the other and spaced from each other. Therein, the X-ray phase modifying unit and the X-ray absorbing unit are arranged within the X-ray beam path in a region upstream of the observation volume. The X-ray phase modifying unit and/or the X-ray absorbing unit comprises at least one sub-unit with periodical sub-structure or preferably a multiplicity of sub-units. Therein, at least one sub-unit or preferably each of the sub-units comprises a steering means for displacing the sub-unit with respect to the other one of the X-ray phase modifying unit and the X-ray absorbing unit. The control device is adapted to controlling the steering means.

According to a second aspect of the invention, a method of operating an X-ray imaging device according to the above-mentioned first aspect is proposed. The method comprises, inter alia, controlling each of the steering means such as to displace the sub-unit(s) of one of the periodical X-ray phase modifying unit and / or the X-ray absorbing unit with respect to the other one of the X-ray phase modifying unit and the X-ray absorbing unit.

For example, according to an embodiment, the control device is adapted to controlling the steering means based on information received from the X-ray detector. Accordingly, based on such X-ray detector information, it may be determined whether specific regions or portions of the X-ray detector tend to be overexposed or underexposed and the controllable transmission arrangement may be controlled accordingly in order to adapt local X-ray transmittance.

For example, according to an embodiment, the control device may be adapted to controlling each of the steering means based on information about accumulated X-ray intensity received by the X-ray detector in a pixel area associated to a respective sub-grating.

According to an embodiment, each sub-unit extends within a plane and each of the steering means is adapted for displacing an associated sub-unit in a direction within this plane. In other words, for example in an embodiment where the sub-units are provided with sub-gratings, the steering means should be adapted for displacing e.g. a planar sub-grating in a lateral direction. By moving the associated grating laterally, the units comprised in the controllable transmission arrangement may be arranged such that the interference patterns generated by a grating forming the periodical X-ray phase modifying unit are positioned in a desired manner with respect to the X-ray absorbing unit created by the third grating, thereby enabling controlling of the transmitted X-ray intensity.

According to an embodiment, the steering means may be adapted for displacing the sub-units using forces induced by electric fields. Based on such electric fields, forces may be generated very rapidly such that a steering means may displace an associated sub-unit very rapidly. For example, displacement rates of up to 5 kHz may be achieved. For example, the steering means may be driven using piezo-elements. Generally, steering means may operate similarly as DLP (Digital Light Processing) devices, using electric forces acting on micro mechanical movable elements.

It shall be noted that various possible features and advantages of embodiments of the invention are described herein partly with respect to an X-ray imaging device and partly with respect to a method of operating such device. One skilled in the art will realize that the various features may be combined, exchanged or replaced throughout the described embodiments in suitable ways and particularly features described with respect to the device may be applied to the method, and vice versa, in analog manners, as far as they fall under the scope of the appended claims.

### BRIEF DESCRIPTION OF THE DRAWINGS

In the following, embodiments of the present invention will be described with respect to the enclosed drawings. However, neither the description nor the drawings shall be interpreted as limiting the scope of the invention.
Fig. 1 shows an X-ray imaging device according to an embodiment of the present invention.
Figs. 2, 3 and 4 show first, second and third gratings, respectively, for an X-ray imaging device according to an embodiment of the present invention.
Fig. 5 shows a multiplicity of sub-gratings for use in an X-ray imaging device according to an embodiment of the present invention.
Fig. 6 illustrates an arrangement of second and third gratings together with created X-ray interference pattern intensities in an X-ray imaging device according to an embodiment of the present invention.

The figures are only schematical and not to scale. Generally, same reference signs are used for same or similar features throughout the figures.

### DETAILED DESCRIPTION OF EMBODIMENTS

Without limiting the scope of the invention in any way, concepts underlying embodiments of the present invention may be understood as being based on the following ideas and recognitions:
In the proposed X-ray imaging device, a controllable transmission arrangement comprises at least two units such as a X-ray phase modifying unit, consisting of at least one sub-unit with periodical structures which generate an interference pattern for X-radiation and an X-ray absorbing unit consisting of at least one sub-unit with periodical structures, which are matched with the interference pattern and attenuate the X-radiation. In embodiments, each of these two units may comprise a grating wherein the gratings are arranged serially within an X-ray beam path and may be used to controllably attenuate a local X-ray photon flux emitted by the X-ray source before reaching the observation volume in which for example a body part of a patient is arranged. Therein, the gratings may be arranged similarly, but may be operated differently, as in a Talbot-Laue interferometer. An optional first grating being for example part of the X-ray source and being e.g. arranged closest to an X-ray tube may be adapted and serve as a source grating. Such source grating may comprise an, e.g. periodic, pattern of highly X-ray absorbing regions and less X-ray absorbing regions. X-rays coming from the X-ray tube and being transmitted through such source grating will be influenced such that the source grating creates an array of individual coherent X-ray sources. Alternatively, a coherent X-ray source such as a synchrotron or small dimensioned coherent X-ray emitting spots may be used. A second grating which is arranged downstream of the first grating is adapted and may serve as a phase grating. Such phase grating generally shows minor X-ray absorbance but comprises a pattern of regions differently influencing a phase of transmitted X-rays. The key characteristic of this device is the, at least local, periodic alteration of the phase of the X-ray waves. Usually it consists of an etched sheet of silicon. But, it may also have a fluid nature or a combination of fluid and gaseous nature. Accordingly, when being transmitted through the phase grating, coherent X-rays form an interference pattern downstream of the phase grating. A third grating which is arranged downstream of the second grating is adapted as and operates as an absorber grating. Such absorber grating comprises a pattern of different regions having different X-ray absorbance similarly as in the source grating.

A general idea underlying such embodiment is to enable relative displacement of the second grating with respect to the third grating using steering means such that the second and third gratings may be displaceably arranged with respect to each other either in a relative position where an interference pattern downstream of the second grating is mainly blocked by the third pattern or, alternatively, in a relative position where such interference X-ray pattern is mainly transmitted through the third grating.

In other words, by enabling relative displacement of the second and third gratings with respect to each other, X-ray transmittance through the entire arrangement of the three gratings may be controlled.

Therein, in order to not only allow influencing an overall X-ray transmittance through the region of interest, at least one of the second and third gratings is divided into a multiplicity of sub-gratings wherein each sub-grating may be independently displaced using an associated steering means. Accordingly, for each area corresponding to a sub-grating, X-ray transmittance may be controlled independently by suitably controlling the associated steering means. If a fluid phase grating is being used, the steering means may be a device that alters the phase shift by creating locally periodic regions of higher and smaller shase shift, including generation of gaseous regions or even vacuum regions.

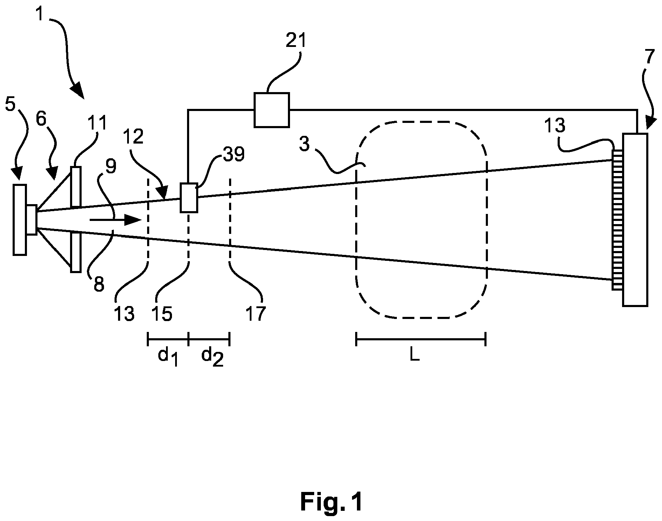

Fig. 1 shows an X-ray imaging device 1 according to an embodiment of the present invention. The X-ray imaging device is adapted for imaging a region of interest of an object arranged within an observation volume 3. For medical applications, such region of interest may be a portion of a human body or animal body. Accordingly, the observation volume 3 may have sufficient dimensions in order to accommodate such body parts. For example, the observation volume 3 may have a length 1 of between 10 and 50 cm, preferably between 30 and 50 cm. The X-ray imaging device 1 comprises an X-ray tube 5 being part of an X-ray source 6 and an X-ray detector 7. Furthermore, the X-ray imaging device 1 comprises an aperture 11, a first grating 13 and a controllable transmission arrangement 12 including a second grating 15 and a third grating 17.

The X-ray source 6 may comprise a conventional X-ray tube 5. The X-ray source 6 is adapted for emitting an X-ray beam 8 along a beam path 9 in a direction towards the X-ray detector 7. The aperture 11 limits the X-ray beam 8 to a certain angle. The path 9 of the X-ray beam traverses the observation volume 3 before reaching a detection surface of the X-ray detector 7. This detection surface may comprise a multiplicity of detector elements arranged in a two-dimensional matrix, wherein these detector elements are also referred to as pixels 19. It may be mentioned that, in alternative embodiments, different X-ray detectors may be used such as for example line detectors in which multiple detector elements or pixels are arranged along a line, i.e. one-dimensionally, and which are typically operated in a scanning manner.

The first, second and third gratings 13, 15, 17 are arranged serially within the beam path 9, i.e. one behind the other, such that the X-ray beam 8 first transmit through the first grating 13, then through the second grating 15 and finally through the third grating 17. Therein, the gratings 13, 15, 17 are arranged spaced from each other, i.e. they have no direct mechanical contact to each other. For example, the second grating 15 may be spaced from the first grating 13 by a distance d₁ of for example between 5 and 50 cm, preferably between 5 and 20cm. The third grating 17 may be arranged at a distance d₂ of for example between 5 and 50 cm, preferably between 5 and 20cm, with respect to the second grating 15. Generally, the distances d₁ and d₂ are significantly smaller than the length 1 of the observation volume 3.

An arrangement comprising the first, second and third gratings 13, 15, 17 may be similar to an arrangement of gratings within a Talbot-Laue interferometer. However, as will be described in further detail below, the arrangement 12 of gratings 13, 15, 17 will be used and operate differently than in a Talbot-Laue interferometer. One main difference may be seen in the fact that all of the first, second and third gratings 13, 15, 17 are arranged upstream of the observation volume 3 whereas in a Talbot-Laue interferometer, a first grating is generally arranged upstream of an observation volume whereas two further gratings are arranged downstream of such observation volume. While details of a Talbot-Laue interferometer shall not be discussed within the present description, reference may be made to Pfeiffer et al.: "Phase retrieval and differential phase-contrast imaging with low-brilliance X-ray sources", Nature Physics, volume 2, April 2006.

Generally, a grating may be understood as a structure with a repetitive pattern of areas with significantly differing properties. For example, such areas may differ with respect to X-ray absorbance thereby enabling influencing a transmission of an X-ray beam through the grating. Alternatively or in addition, the areas may differ with respect to X-ray propagation velocity thereby enabling influencing phase characteristics of an X-ray beam through the grating. Generally the gratings 13, 15, 17 described herein may have structural dimensions in a micrometer range, with structures or a periodicity of the gratings being e.g. less than 50µm, preferably less than 20µm or less than 10µm.

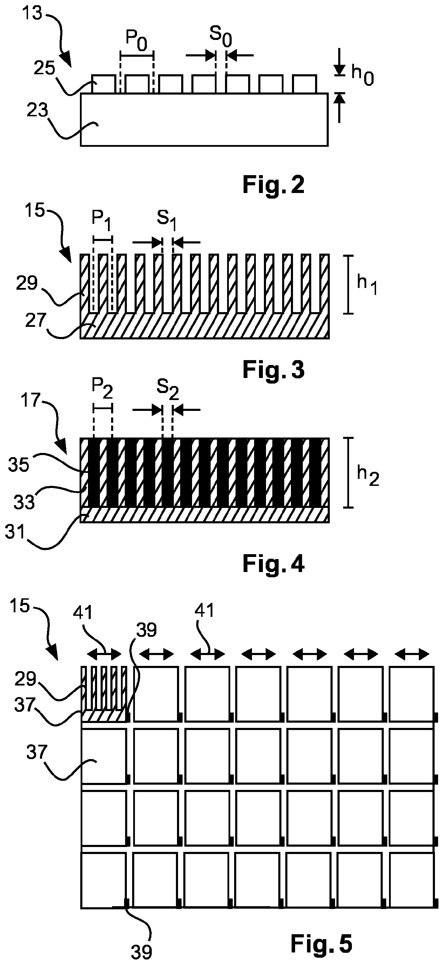

Fig. 2 shows an example of a first grating 13. This first grating 13 comprises a base substrate 23 of e.g. silicon. At a surface of this base substrate 23, a multiplicity of rectangular blocks 25 is arranged in a periodically repetitive pattern. Each of the blocks 25 typically comprises a highly X-ray absorbing material such as e.g. gold. A periodical length or periodicity p₀ may be between 10 and 50 micrometers. Neighboring blocks 25 are spaced from each other by a distance or spacing s₀ being in a range of 1 to 10 micrometers. A height h₀ of the blocks 25 may be in a range of 10 to 200 micrometers. In the arrangement 12, such first grating 13 may serve as a source grating, being sometimes also referred to "G₀". When such source grating is illuminated by a generally non-coherent X-ray beam 8, it creates an array of individually coherent source X-ray beams with superimposed patterns of destructive and constructive interference each. In other words, even when the X-ray source 6 of the X-ray imaging device 1 does not produce coherent X-ray radiation, after transmission through the first grating 13, the X-radiation comprises a certain degree of spatial coherence which is important to generate interference patterns by the subsequent second grating 15.

An example of a second grating 15 is shown in Fig. 3. The second grating 15 also has a basis 27 and some blocks 29. Contrary to the first grating 13 in which the basis 23 and the blocks 25 typically consist of different materials, the base 27 and blocks 29 of the second grating 15 typically consist of a same material such as for example silicon. The second grating 15 may be made as a single piece. The blocks 29 are formed as elongate fingers extending from the base 27 and having a height h₁ of between 1 and 20 micrometers. A periodical length p₁ may be in a range of 1 to 10 micrometers and a spacing s₁ between neighboring blocks 29 may be in a range of 1 to 10 micrometers. Typically, the blocks 29 forming the periodical structure of the second grating 15 consist of a weakly X-ray absorbing material such as silicon. Accordingly, X-rays transmitted through such second grating 15 are not substantially absorbed. However, such transmitted X-rays are subject to different propagation velocities depending on whether they are transmitted through the blocks 29 or through the intermediate spacings between neighboring blocks 29. Accordingly, the second grating 15 may act as a phase grating. When coherent X-radiation is transmitted through such phase grating, interference patterns are formed downstream of the second grating 15.

An example of a third grating 17 is shown in Fig. 4. This third grating again comprises a base substrate 31 of e.g. silicon and a periodical pattern of blocks on top of this base substrate 31. As shown in the example of Fig. 4, such periodical pattern comprises first blocks 33 and second blocks 35 wherein the first and second blocks 33, 35 are arranged alternately. The blocks 33, 35 are formed like elongate fingers with a height h₂ of between 10 and 200 micrometers. The pattern comprises a periodical length p₂ of between 1 and 10 micrometers. A thickness of the first blocks 33 is between 1 and 10 micrometers wherein such thickness may also be interpreted as spacing s₂ between neighboring second blocks 35. The second blocks 35 comprise a highly X-ray absorbing material such as e.g. gold. The first blocks 33 generally comprise a weakly X-ray absorbing material such as e.g. silicon. Due to the highly X-ray absorbing second blocks 35, the third grating 17 may act as an absorber grating having a periodical pattern of highly X-ray absorbing second blocks 35.

A purpose of such third grating 17 or absorber grating within the arrangement 12 may be seen in enabling selectively and controllingly transmitting X-rays which, after having passed the second grating 15 or phase grating, arrive at the third grating 17 with an interference pattern having a certain periodicity pᵢ. Generally, the periodicity pᵢ of the interference pattern depends on the periodicity p₁ of the second grating 15 as well as on the distance d₂ between the second grating 15 and the third grating 17. Accordingly, it may be advantageous to provide the third grating 17 with a periodicity p₂ substantially corresponding to the periodicity pᵢ of the interference pattern at the location of the third grating 17.

With a geometry having such periodicity p₂, the third grating 17 may either be arranged such that regions of maximum X-ray intensity within the interference pattern coincide with the highly X-ray absorbing second blocks 35 and are therefore strongly attenuated, or such that they coincide with the weakly X-ray absorbing first blocks 33 and then they are only weakly attenuated upon transmission through the third grating 17.

Accordingly, whether or not the X-ray beam 8 is strongly attenuated upon transmission through the arrangement 12 of first, second and third gratings 13, 15, 17 mainly depends on the relative positioning of the second and third gratings 15, 17.

It may be noted that each of the gratings 13, 15, 17 and their sub-gratings may be manufactured using various manufacturing techniques. For example, the gratings may be manufactured as arrays of micrometer sized MEMS (micro electro-mechanical systems) structures. Such MEMS structures may be fabricated using for example approved silicon manufacturing technologies such as photolithography and selective etching processes.

Furthermore, it may be noted that, while the first grating 13 is shown with strongly X-ray absorbing blocks 25 only and the third grating 17 is shown with both strongly X-ray absorbing blocks 35 as well as weakly X-ray absorbing blocks 33, each of these first and third gratings 17 may or may not have weakly X-ray absorbing blocks. For example, such weakly X-ray absorbing blocks may be formed in a silicon substrate and spaces between neighboring silicon fingers may then be filled with a highly X-ray absorbing material such as gold, thereby forming the X-ray absorbing blocks 25, 35.

Fig. 5 shows an example of a second grating 15 which may be used within the arrangement 12 of an X-ray imaging device 1 according to an embodiment of the present invention. The second grating 15 comprises a multiplicity of sub-gratings 37. The sub-gratings 37 are arranged in a 2-dimensional matrix. Each of the sub-gratings 37 may comprise a geometry similar to the geometry shown in Fig. 3 with blocks 29 of a weakly X-ray absorbing but phase-delaying material being arranged in a periodical pattern. In Fig. 5, such periodical pattern is only shown exemplarily for the upper left sub-grating 37. In the second grating 15, each sub-grating 37 comprises an associated steering means 39. This steering means 39 is adapted to displacing the associated sub-grating 37. For example, the steering means 39 may use forces induced by electric fields for displacing the sub-grating 37. For example, piezoelectric crystals may be used as steering means 39.

Therein, the steering means 39 may be adapted and arranged such that it displaces the associated sub-grating 37 laterally as shown with the arrows 41, i.e. within a plane of the planar sub-grating 37 and preferably in a direction of the periodicity of the sub-grating 37, i.e. transverse to a direction of the fingers of the blocks 29. Thus, using the means steering means 39, each of the sub-gratings 37 may be controllably displaced within the plane of the second grating 15 and may therefore be displaced relative to the neighboring third grating 17.

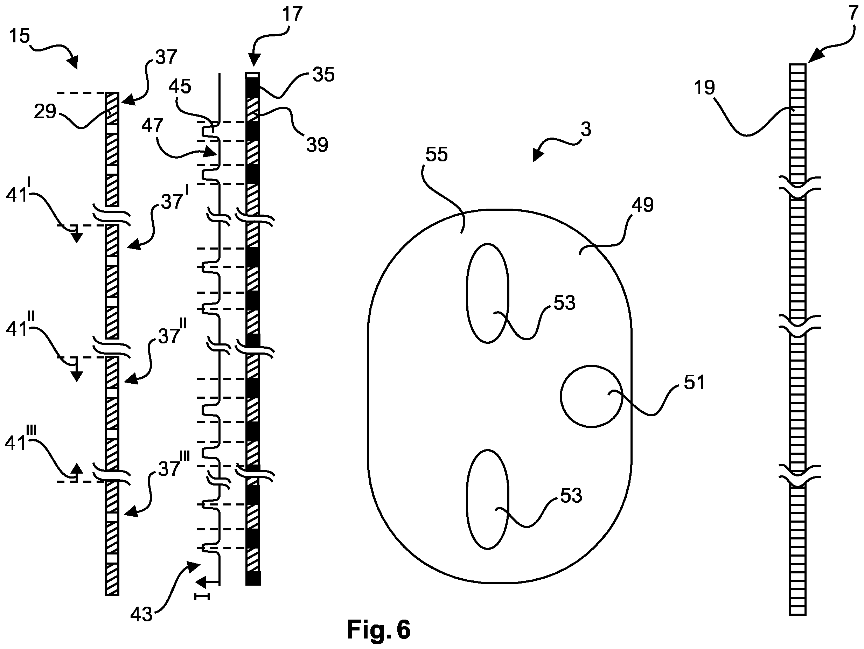

An operating principle underlying embodiments of the presented X-ray imaging device 1 as well as an exemplary method for operating such device will be explained in further detail with reference to Fig. 6. An X-ray beam 8 originating from the X-ray source 6 and being transmitted through the first grating 13 (not shown in fig. 6) arrives at the second grating 15 with a sufficient degree of spatial coherence. The second grating 15 then acts as a phase grating due to its periodical pattern. Accordingly, upon transmission through such phase grating, an interference pattern will form within the X-ray beam 8 downstream of the second grating 15.

A distribution of X-ray intensity I within such interference pattern behind the second grating 15 is schematically shown in Fig. 6 as a graph 43. The intensity distribution in the interference pattern shows a periodical arrangement of intensity maxima 45 with intermediate intensity minima 47. The exact location and size of the intensity maxima 45 and intensity minima 47 mainly depends on the positioning and periodicity p₁ of the periodical structures within the second grating 15 and the distance d₂ between the second and third gratings 15, 17.

As explained above with reference to Fig. 5, the second grating 15 is divided into a multiplicity of sub-gratings 37 and each sub-grating 37 may be displaced using a steering means 39 (not shown in Fig. 6) in a lateral direction 41.

Accordingly, each of a multiplicity of sub-gratings 37, 37', 37", 37"' may be specifically positioned such that an interference pattern generated upon X-ray transmission through the respective sub-grating is created such that its intensity maxima 45 spatially coincide with the highly absorbing blocks 35 of the subsequent third grating 17 (as shown for the uppest sub-grating 37 in Fig. 6) or the intensity maxima 45 spatially coincide with the weakly intermediate blocks 33 (as shown in the second lowest sub-grating 37"). Of course, intermediate positioning in which the intensity maxima 45 partly overlap with highly absorbing blocks 35, as shown for the other sub-gratings 37', 37"', may also be chosen. In order to specifically control the local transmission characteristics of the arrangement 12 of gratings 13, 15, 17, the means 39 may be controlled based on information received from the X-ray detector 7.

For example, as shown in Fig. 6, a human body 49 may be arranged within the observation volume 3. Such human body 49 comprises highly X-ray absorbing portions such as a backbone 51 as well as weakly X-ray absorbing regions comprising for example lung tissue 53. Furthermore, the body 49 may comprise peripheral regions 55 which are of no interest during medical X-ray imaging.

Accordingly, in order to minimize an X-ray dose supplied to the patient and, at the same time, avoid overexposure and/or underexposure of regions of the X-ray detector 7, information about accumulated X-ray intensity received by the X-ray detector in an area comprising one or more pixels 19 associated to one of the respective sub-gratings 37, 37', 37", 37"' of the second grating 15 may be used in order to specifically position this sub-grating 37, 37', 37", 37"'.

E.g. in the example shown in Fig. 6, the uppermost sub-grating 37 is positioned such that maxima 45 of the interference pattern coincide with the highly X-ray absorbing blocks 35 such that substantially no X-rays are transmitted to the peripheral and non-interesting areas 55 of the body 49.

All other sub-gratings 37', 37", 37'" may first be arranged such that a maximum intensity of X-rays is transmitted through the arrangement 12 of gratings 13, 15, 17 and X-ray intensity is accumulated within the pixels 19of the detector 7. The information about the accumulated X-ray intensity is continuously monitored and as soon as it is recognized that partial areas of the detector 7 tend to be over-exposed, such information may be used in a control device 21 (see fig. 1) which is connected to the detector 7 and to each of the steering means 39 of the second grating 15.

The control device 21 may then control steering means 39 of sub-gratings 37', 37"' associated to the nearly overexposed detector portions such that at least part of the intensity maxima 45 spatially coincide with the highly absorbing blocks 35 of the third grating 17 and therefore portions of the X-ray beam 8 are blocked in the respective areas.

In contrast hereto, areas of the detector 7 downstream of highly X-ray absorbing structures such as the backbone 51 within the body 49 will not tend to be overexposed and thus the control device 21 will control the steering means 39 of an associated sub-grating 37" such that intensity maxima 45 of a generated interference pattern coincide with the weakly absorbing blocks 33 of the third grating 17 and are therefore maximally transmitted.

It may be noted that in order to be able to control X-ray transmittance through the arrangement 12, it may be only necessary to be able to control the relative positioning of the second and third gratings. Accordingly, in principle, instead of or in addition to moving sub-gratings of the second grating 15, the third grating 17 or sub-gratings thereof may be actively moved using steering means.

However, it may generally be easier to move sub-gratings 37 of the second grating 15 as these sub-gratings 37 are typically lighter than optional sub-gratings of the third grating 17 as this third grating 17 comprises highly X-ray absorbing blocks 35 of heavy materials such as gold.

In a specific embodiment of the presented X-ray imaging device 1, the X-ray source 6 may be adapted to emit a poly-chromatic spectrum of X-rays. In such embodiment, the control device 21 may be adapted to controlling the steering means 39 such that, during an X-ray imaging acquisition, sub-gratings 37 are displaced such that varying X-ray spectra are transmitted through the arrangement 12 of first, second and third gratings13, 15, 17. Such embodiment may be seen as based on the idea that a Talbot-pattern is mainly dependent on a wavelength of transmitted X-rays and therefore attenuation of X-rays within the pre-patient arrangement 12 of gratings 13, 15, 17 is generally maximal only for a specific ("design-") wavelength and grating setting. E.g., when using a poly-chromatic X-ray beam, photons with other wavelengths will at least partly pass the arrangement 12. Accordingly, by modulating the grating structure and positioning as explained above, a specific object of interest such as e.g. a vessel structure within a patient may be illuminated with photons of different mean energy.

In a further specific embodiment, the periodical X-ray phase modifying unit 15 comprises an array of structures or gratings such as e.g. sub-millimeter sized pieces of etched silicon. Such array may comprise e.g. one or several sources of ultrasound waves. For example, such sources may be provided using modern cMut-sources which may be formed in a MEMS as an array of ultrasound sources. Thus, the array may be attached to localized ultrasound transducers. Such array may be immersed in a liquid. Upon activation of the localized transducers, a periodical pattern of cavitations may be generated in the liquid, which may act as localized periodic elements to alter or modify a phase shift in transmitted X-rays. In other words, the cavitations generally comprise a different index of refraction compared to surrounding liquid such that a period pattern of refraction index changes is created. Due to the phase shift occurring upon transmission of X-rays through such pattern, an interference pattern may form and beam intensity behind the array will be altered locally. With the periodical X-ray absorbing unit 17 being arranged specifically with respect to such interference pattern, the intensity of X-rays transmitted through the entire controllable transmission arrangement 12 may be controlled.

### LIST OF REFERENCE SIGNS:

- 1: X-ray imaging device
- 3: observation volume
- 5: X-ray tube
- 6: X-ray source
- 7: X-ray detector
- 8: X-ray beam
- 9: beam path
- 11: aperture
- 12: arrangement
- 13: first grating
- 15: second grating
- 17: third grating
- 19: pixel
- 21: control device
- 23: base substrate of first grating
- 25: X-ray absorbing block of first grating
- 27: base substrate of second grating
- 29: X-ray phase changing blocks of second grating
- 31: base substrate of third grating
- 33: X-ray phase changing blocks of third grating
- 35: X-ray absorbing block of third grating
- 37: sub-grating
- 39: steering means
- 41: displacement direction
- 43: graph indicating X-ray intensity distribution
- 45: X-ray intensity maxima
- 47: X-ray intensity minima
- 49: body
- 51: backbone
- 53: lung tissue
- 55: peripheral area

## Claims

1. X-ray imaging device (1) for imaging a region of interest of an object (49) arranged within an observation volume (3), the X-ray imaging device comprising:
an X-ray source (5) adapted for generating at least partially coherent X-radiation, a controllable transmission arrangement (12) comprising an at least locally periodical X-ray phase modifying unit (15) for generating an interference pattern when the at least partially coherent X-ray radiation is transmitted through it, and an at least locally periodical X-ray absorbing unit (17) which is arranged downstream of the phase modifying unit,
an X-ray detector (7), and
a control device (21),
wherein the periodical X-ray phase modifying unit and the periodical X-ray absorbing unit are arranged behind each other and spaced from each other in a beam path (9) of X-rays emitted by the X-ray source in a region upstream of the observation volume;
wherein at least one of the periodical X-ray phase modifying unit and the periodical X-ray absorbing unit comprises a multiplicity of sub-units (37), wherein at least one of the sub-units comprises steering means (39) for displacing the associated sub-units, wherein a control device is adapted to controlling the at least one steering means.

2. X-ray imaging device according to claim 1, wherein the control device is adapted to controlling the steering means based on information received from the X-ray detector.

3. X-ray imaging device according to one of claims 1 and 2, wherein the periodical X-ray phase modifying unit is a phase grating (15) and the periodical X-ray absorbing unit is an absorber grating (17), the absorber grating comprising a repetitive pattern of areas with significantly differing X-ray absorbance, and the phase grating comprising a repetitive pattern of areas differently influencing a phase of transmitted X-rays.

4. X-ray imaging device according to claim 1, wherein the X-ray source comprises a source grating (13).

5. X-ray imaging device according to claim 3 and 4, wherein the source, phase and absorber gratings comprise repetitive patterns with a periodicity p₀, p₁, p₂ of less than 50 micrometers.

6. X-ray imaging device according to claim 3, wherein at least one of the phase and absorber gratings is divided into a multiplicity of sub-gratings (37, 37', 37", 37"'), wherein each sub-grating may be independently displaced using an associated steering means (39).

7. X-ray imaging device according to claims 2 and 6, wherein the control device is adapted to controlling each of the steering means based on information about accumulated X-ray intensity received by the X-ray detector in a pixel area associated to the respective sub-grating.

8. X-ray imaging device according to one of claims 1 or 6, wherein each sub-unit or sub-grating extends within a plane and wherein the steering means are adapted for displacing the sub-units or sub-gratings in a direction (41) laterally within the plane.

9. X-ray imaging device according to claim 1, wherein the steering means are adapted for displacing the sub-units using forces induced by electric fields.

10. X-ray imaging device according to claim 1, wherein the sub-units are arranged in a 2-dimensional matrix.

11. X-ray imaging device according to claim 1, wherein the X-ray source is adapted to emit a polychromatic spectrum of X-rays and wherein the control device is adapted to controlling the steering means such that during an X-ray image acquisition sub-units are displaced such that varying X-ray spectra are transmitted through the controllable transmission arrangement.

12. X-ray imaging device according to claim 1, wherein the periodical X-ray phase modifying unit comprises means for generating an at least local periodical pattern of cavitations within a liquid.

13. Method of operating an X-ray imaging device (1) according to claim 1, comprising:
controlling each of the steering means (39) such as to alter the interference pattern generated by at least one of the sub-units (37) of the X-ray phase modifying unit with respect to the X-ray absorbing unit.

14. Method according to claim 13, wherein the steering means are controlled based on information about accumulated X-ray intensity received by the X-ray detector (7) in a pixel area associated to the respective sub-units.

15. Method according to one of claims 13 and 14, wherein the X-ray source (5) is adapted to emit a polychromatic spectrum of X-rays and wherein the method further comprises controlling the steering means such that during an X-ray image acquisition sub-gratings are displaced such that varying X-ray spectra are transmitted through the controllable transmission arrangement (12).

## Patentansprüche

1. Röntgenbildgebungsvorrichtung (1) zur Bildgebung einer interessierenden Region eines Objekts (49), das in einem Untersuchungsvolumen (3) angeordnet ist, wobei die Röntgenbildgebungsvorrichtung Folgendes umfasst:
eine Röntgenquelle (5), die dafür vorgesehen ist, mindestens teilweise kohärente Röntgenstrahlung zu erzeugen,
eine steuerbare Übertragungsanordnung (12) umfassend eine mindestens lokal periodische Röntgenphasenmodifizierungseinheit (15) zum Erzeugen eines Interferenzmusters, wenn die mindestens teilweise kohärente Röntgenstrahlung durch diese gesendet wird, und eine mindestens lokal periodische Röntgenabsorbierungseinheit (17), die flussabwärts der Phasenmodifizierungseinheit angeordnet ist,
einen Röntgendetektor (7) und
eine Steuervorrichtung (21),
wobei die periodische Röntgenphasenmodifizierungseinheit und die periodische Röntgenabsorbierungseinheit hintereinander und beabstandet voneinander in einem Strahlenbündelpfad (9) von durch die Röntgenquelle emittierten Strahlen in einer Region flussaufwärts des Untersuchungsvolumens angeordnet sind;
wobei mindestens entweder die periodische
Röntgenphasenmodifizierungseinheit oder die periodische Röntgenabsorbierungseinheit eine Vielzahl von Subeinheiten (37) umfasst, wobei mindestens eine der Subeinheiten Lenkungsmittel (39) zum Bewegen der zugehörigen Subeinheiten umfasst,
wobei eine Steuervorrichtung dafür vorgesehen ist, das mindestens eine Lenkungsmittel zu steuern.

2. Röntgenbildgebungsvorrichtung nach Anspruch 1, wobei die Steuervorrichtung dafür vorgesehen ist, die Lenkungsmittel basierend auf von dem Röntgendetektor empfangenen Informationen zu steuern.

3. Röntgenbildgebungsvorrichtung nach einem der Ansprüche 1 und 2, wobei die periodische Röntgenphasenmodifizierungseinheit ein Phasengitter (15) ist und die periodische Röntgenabsorbierungseinheit ein Absorbergitter (17) ist, wobei das Absorbergitter ein sich wiederholendes Muster von Bereichen mit signifikant unterschiedlicher Röntgenabsorption umfasst und das Phasengitter ein sich wiederholendes Muster von Bereichen umfasst, die eine Phase von ausgesendeten Röntgenstrahlen unterschiedlich beeinflussen.

4. Röntgenbildgebungsvorrichtung nach Anspruch 1, wobei die Röntgenquelle ein Quellengitter (13) umfasst.

5. Röntgenbildgebungsvorrichtung nach den Ansprüchen 3 und 4, wobei das Quellen-, das Phasen- und das Absorbergitter sich wiederholende Muster mit einer Periodizität p₀, p₁, p₂ von weniger als 50 Mikrometer umfassen.

6. Röntgenbildgebungsvorrichtung nach Anspruch 3, wobei mindestens entweder das Phasen- oder das Absorbergitter in eine Vielzahl von Subgittern (37, 37', 37", 37"') unterteilt ist, wobei jedes Subgitter unter Verwendung eines zugehörigen Lenkungsmittels (39) unabhängig bewegt werden kann.

7. Röntgenbildgebungsvorrichtung nach den Ansprüchen 2 und 6, wobei die Steuervorrichtung dafür vorgesehen ist, jedes der Lenkungsmittel basierend auf Informationen über angesammelte Röntgenintensität zu steuern, die durch den Röntgendetektor in einem zu dem jeweiligen Subgitter gehörenden Pixelbereich empfangen werden.

8. Röntgenbildgebungsvorrichtung nach einem der Ansprüche 1 oder 6, wobei jede Subeinheit oder jedes Subgitter sich in einer Ebene erstreckt und wobei die Lenkungsmittel dafür vorgesehen sind, die Subeinheiten oder Subgitter in einer lateralen Richtung (41) in der Ebene zu bewegen.

9. Röntgenbildgebungsvorrichtung nach Anspruch 1, wobei die Lenkungsmittel dafür vorgesehen sind, die Subeinheiten unter Verwendung von durch elektrische Felder induzierten Kräften zu bewegen.

10. Röntgenbildgebungsvorrichtung nach Anspruch 1, wobei die Subeinheiten in einer zweidimensionalen Matrix angeordnet sind.

11. Röntgenbildgebungsvorrichtung nach Anspruch 1, wobei die Röntgenquelle dafür vorgesehen ist, ein polychromatisches Röntgenstrahlenspektrum zu emittieren, und wobei die Steuervorrichtung dafür vorgesehen ist, die Lenkungsmittel so zu steuern, dass Subeinheiten während der Erfassung eines Röntgenbilds derartig bewegt werden, dass variierende Röntgenspektren durch die steuerbare Übertragungsanordnung übertragen werden.

12. Röntgenbildgebungsvorrichtung nach Anspruch 1, wobei die periodische Röntgenphasenmodifizierungseinheit Mittel zum Erzeugen eines mindestens lokal periodischen Musters von Kavitationen in einer Flüssigkeit umfasst.

13. Verfahren zum Betreiben einer Röntgenbildgebungsvorrichtung (1) nach Anspruch 1, umfassend:
Steuern von jedem der Lenkungsmittel (39) auf derartige Weise, dass das durch mindestens eine der Subeinheiten (37) der Röntgenphasenmodifizierungseinheit erzeugte Interferenzmuster in Bezug auf die Röntgenabsorbierungseinheit geändert wird.

14. Verfahren nach Anspruch 13, wobei die Lenkungsmittel basierend auf Informationen über angesammelte Röntgenintensität gesteuert werden, die durch den Röntgendetektor (7) in einem zu den jeweiligen Subeinheiten gehörigen Pixelbereich empfangen werden.

15. Verfahren nach einem der Ansprüche 13 und 14, wobei die Röntgenquelle (5) dafür vorgesehen ist, ein polychromatisches Röntgenspektrum zu emittieren, und wobei das Verfahren weiterhin das Steuern der Lenkungsmittel auf derartige Weise umfasst, dass während der Erfassung eines Röntgenbilds Subgitter derartig bewegt werden, dass variierende Röntgenspektren durch die steuerbare Übertragungsanordnung (12) übertragen werden.

## Revendications

1. Dispositif d'imagerie à rayons X (1) permettant d'imager une région d'intérêt d'un objet (49) placé au sein d'un volume d'observation (3), le dispositif d'imagerie à rayons X comprenant :
une source de rayons X (5) adaptée pour générer un rayonnement X au moins partiellement cohérent,
un agencement de transmission contrôlable (12) comprenant une unité de modification de phase de rayons X au moins localement périodique (15) pour générer un modèle d'interférence lorsque le rayonnement de rayons X au moins partiellement cohérent est transmis à travers elle, et une unité d'absorption de rayons X au moins localement périodique (17) qui est placée en aval de l'unité de modification de phase,
un détecteur de rayons X (7), et
un dispositif de commande (21),
dans lequel l'unité de modification de phase de rayons X périodique et l'unité d'absorption de rayons X périodique sont placées l'une derrière l'autre et éloignées l'une de l'autre dans un faisceau canalisé (9) de rayons X émis par la source de rayons X dans une région en amont du volume d'observation ;
dans lequel au moins l'une de l'unité de modification de phase de rayons X périodique et l'unité d'absorption de rayons X périodique comprend une multiplicité de sous-unités (37), dans lequel au moins l'une des sous-unités comprend des moyens d'orientation (39) permettant de déplacer les sous-unités associées,
dans lequel un dispositif de commande est adapté pour commander les au moins un moyens d'orientation.

2. Dispositif d'imagerie à rayons X selon la revendication 1, dans lequel le dispositif de commande est adapté pour commander les moyens d'orientation sur la base d'informations reçues du détecteur de rayons X.

3. Dispositif d'imagerie à rayons X selon l'une des revendications 1 et 2, dans lequel l'unité de modification de phase de rayons X périodique est un réseau de phase (15) et l'unité d'absorption de rayons X périodique est un réseau d'absorption (17), le réseau d'absorption comprenant un modèle répétitif de zones à absorbance des rayons X variant sensiblement, et le réseau de phase comprenant un modèle répétitif de zones influençant de manière différente une phase de rayons X transmis.

4. Dispositif d'imagerie à rayons X selon la revendication 1, dans lequel la source de rayons X comprend un réseau de source (13).

5. Dispositif d'imagerie à rayons X selon la revendication 3 et 4, dans lequel les réseaux de source, de phase et d'absorption comprennent des modèles répétitifs d'une périodicité p₀, p₁, p₂ inférieure à 50 micromètres.

6. Dispositif d'imagerie à rayons X selon la revendication 3, dans lequel au moins l'un des réseaux de phase et d'absorption est divisé en une multiplicité de sous-réseaux (37, 37', 37", 37"'), dans lequel chaque sous-réseau peut être déplacé indépendamment à l'aide des moyens d'orientation associés (39).

7. Dispositif d'imagerie à rayons X selon les revendications 2 et 6, dans lequel le dispositif de commande est adapté pour commander chacun des moyens d'orientation sur la base d'informations concernant l'intensité cumulée des rayons X reçue par le détecteur de rayons X dans une zone de pixels associée au sous-réseau respectif.

8. Dispositif d'imagerie à rayons X selon l'une des revendications 1 ou 6, dans lequel chaque sous-unité ou sous-réseau s'étend dans un plan et dans lequel les moyens d'orientation sont adaptés pour déplacer les sous-unités ou sous-réseaux dans une direction (41) latéralement dans le plan.

9. Dispositif d'imagerie à rayons X selon la revendication 1, dans lequel les moyens d'orientation sont adaptés pour déplacer les sous-unités à l'aide de forces induites par des champs électriques.

10. Dispositif d'imagerie à rayons X selon la revendication 1, dans lequel les sous-unités sont agencées dans une matrice bidimensionnelle.

11. Dispositif d'imagerie à rayons X selon la revendication 1, dans lequel la source de rayons X est adaptée pour émettre un spectre polychromatique de rayons X et dans lequel le dispositif de commande est adapté pour commander les moyens d'orientation de telle sorte qu'au cours d'une acquisition d'image radiologique, les sous-unités sont déplacées de telle sorte que les spectres de rayons X sont transmis par le biais de l'agencement de transmission contrôlable.

12. Dispositif d'imagerie à rayons X selon la revendication 1, dans lequel l'unité de modification de phase de rayons X périodique comprend des moyens permettant de générer un modèle périodique au moins local de cavitations à l'intérieur d'un liquide.

13. Procédé de fonctionnement d'un dispositif d'imagerie à rayons X (1) selon la revendication 1, comprenant :
la commande de chacun des moyens d'orientation (39) de sorte à modifier le modèle d'interférence généré par au moins l'une des sous-unités (37) de l'unité de modification de phase de rayons X par rapport à l'unité d'absorption de rayons X.

14. Procédé selon la revendication 13, dans lequel les moyens d'orientation sont commandés sur la base d'informations concernant l'intensité cumulée des rayons X reçue par le détecteur de rayons X (7) dans une zone de pixels associée aux sous-unités respectives.

15. Procédé selon l'une des revendications 13 et 14, dans lequel la source de rayons X (5) est adaptée pour émettre un spectre polychromatique de rayons X et dans lequel le procédé comprend en outre la commande des moyens d'orientation de sorte qu'au cours d'une acquisition d'image radiologique, les sous-réseaux sont déplacés de telle sorte que des spectres de rayons X variables sont transmis par le biais de l'agencement de transmission contrôlable (12).
